# EUROPÄISCHE PATENTSCHRIFT

(11) **EP 1 009 419 B1**
(45) Veröffentlichungstag und Bekanntmachung des Hinweises auf die Patenterteilung: **24.05.2006**
(21) Anmeldenummer: 98914887.9
(22) Anmeldetag: 16.03.1998
(51) Int. Cl.: C07K 14/415

(54) **Verfahren zur Identifizierung modifizierter rekombinanter Graminaenpollen-Allergene**
Method for identifying modified recombinant Graminae pollen allergens
Méthode pour l'identification d'allergènes recombinants modifiés de pollen de Graminées

(30) Priorität: 27.03.1997 DE 19713001
(43) Veröffentlichungstag der Anmeldung: 21.06.2000
(73) Patentinhaber: Merck Patent GmbH, 64293 Darmstadt (DE)
(72) Erfinder: KAHLERT, Helga, D-22041 Hamburg (DE); STÜWE, Hans-Thomas, D-21435 Stelle (DE); FIEBIG, Helmut, D-21493 Schwarzenbek (DE); CROMWELL, Oliver, D-21465 Wentorf (DE); BECKER, Wolf-Meinhard, D-23975 Mözen (DE); BUFE, Albrecht, D-20255 Hamburg (DE); SCHRAMM, Gabriele, D-23867 Süllfeld (DE); JÄGER, Lothar, D-07743 Jena (DE); MÜLLER, Wolf-Dieter, D-07749 Jena (DE)
(74) Vertreter: Schüttler, Reinhard
(86) Internationale Anmeldenummer: PCT/EP1998/001507
(87) Internationale Veröffentlichungsnummer: WO 1998/043657

(56) Entgegenhaltungen:
- WO-A-91/06571
- WO-A-94/04564
- MÜLLER, W.D. ET AL.: "Group 5 allergens of timothy grass ..." INT. ARCH. ALLERGY IMMUNOL., Bd. 109, 1996, Seiten 352-355, XP002077934
- BECKER, W.M.: "Molekulare Charakterisierung von Allergenen" IMMUN. INFEKT., Bd. 22, 1994, Seiten 82-87, XP002077935

## Beschreibung

Die Erfindung betrifft ein Verfahren zur Identifizierung modifizierter rekombinanter Graminaenpollen-Allergene, die von den Hauptgruppen 1 oder 5 abgeleitet sind, deren Reaktivität mit IgE-Antikörpern von gegen diese Allergene allergischen Patienten eliminiert oder reduziert ist, wobei die Reaktivität mit T-Lymphozyten weiterhin erhalten ist.

Graminaenpollenextrakte, wie sie für diagnostischen und therapeutischen Einsatz verwendet werden, bestehen aus einem heterogenen Gemisch von Proteinen und Glykoproteinen, unter denen einige mit IgE-Antikörpern von Allergikern reagieren und definitionsgemäß als Allergene bezeichnet werden. Die molekularen Eigenschaften erlauben eine Klassifizierung in 6 Gruppen, wobei die Kreuzreaktivität der in Frage kommenden Graminaen-Spezies relativ hoch ist. Die dominierenden Allergengruppen (Hauptallergene) sind die Gruppen 5 und 1, gemäß der üblichen Allergen-Klassifizierung (Liebers et al., Clin. Exper. Allergy, 26, 494-516 (1996)). Die N-terminalen Aminosäuresequenzen und/oder die partiellen oder vollständigen deduzierten Aminosäuresequenzen der Gruppen 5 und 1 der Hauptallergene sind bekannt (Vrtala et al., J. lmmunology 151, 4773-4781 (1993) u. Bufe et al. FEBS. Lett. 263, 6-12 (1995)). Weiterhin gibt es beschriebene Verfahren zur Klonierung dieser Hauptallergene (Scheiner et al. lnt. Arch Allergy Immunol. 98, 93-96 (1992)).

Gegenwärtig werden zur In-vitro-Diagnostik von Typ 1-Allergien wäßrige Extrakte aus Graminaenpollen verwendet. Diese Extrakte sind auch die Basis für die In-vitro-Diagnostik und zur anschließenden spezifischen Immuntherapie (Fiebig H., Allergo Journal Z, 377-382 (1995)). Der Einsatz von nativen Allergenextrakten zur spezifischen Immuntherapie wird durch die dabei induzierten IgE-bedingten, allergischen Reaktionen (Nebenreaktionen) begrenzt. Deshalb können native Allergenextrakte nur in Dosierungen unterhalb der Nebenwirkungsschwelle appliziert werden. Um die für den therapeutischen Effekt notwendigen hohen Allergenkonzentrationen zu erreichen, werden die Extrakte durch mehrfache aufeinanderfolgende Injektionen mit einer bis zur Erhaltungsdosis ansteigenden Konzentration verabreicht. Durch Adsorption an Gele ist es möglich, Allergenextrakte nebenwirkungsärmer und effektiver zur Hyposensibilisierung zu verwenden.

Eine weitere Verbesserung konnte durch die chemische Modifizierung der Allergene zu Allergoiden, die eine verringerte lgE-Reaktivität bei weitgehend erhaltener Immunogenität besitzen, erzielt werden (Fiebig H., Allergo Journal 7, 377-382 (1995) u. Maasch et al. Clin. Ref. Allergy 5, 89-106 (1987)).

In ersten Untersuchungen mit Hausstaubmilbenallergenen gibt es Hinweise, daß durch gerichteten Aminosäureaustausch eine Reduzierung der IgE-Reaktivität erzielt werden kann (Smith et al. Mol. Immunol. 33, 399-405 (1996) u. Nishiyama et al. Mol. Immunol. 32, 1021-1029 (1995)).

Die etablierte Hyposensibilisierung von Graminaenpollenallergikern erfolgt momentan mit natürlichen Extrakten, die alle bekannten Allergene sowie nichtallergene, aber immunogene Begleitstoffe in beträchtlichen Konzentrationen enthalten, obwohl für die allergenspezifische Therapie nur diejenigen Allergenmoleküle benötigt werden, gegen die der jeweilige Patient tatsächlich sensibilisiert ist. Das bedeutet, daß der Allergiker zwangsläufig mit Komponenten behandelt wird, die nicht zu seiner Hyposensibilisierung beitragen und Nebenwirkungen induzieren können.

Durch die Verfügbarkeit von modifizierten rekombinanten Allergenen können einzelne Allergene oder definierte Gemische für die Hyposensibilisierung entsprechend dem individuellen Sensibilisierungsspektrum als Arzneimittel eingesetzt werden.

Daraus ergibt sich die Möglichkeit einer spezifischen, maßgeschneiderten Therapie.

Der Erfindung lag die Aufgabe zugrunde, neue Verfahren zur Identifizierung von Allergenen-Mutanten zur Herstellung von Arzneimitteln aufzufinden.

Überraschenderweise ist es im Rahmen der vorliegenden Erfindung gelungen, ein Verfahren, zur Identifizierung von Mutanten zu entwickeln, die mit T-Lymphozyten von Graspollenallergikern spezifisch reagieren, d.h. zur Proliferation und Zytokinsynthese stimulieren oder eine Anergie induzieren, aber mit den im Serum der T-Lymphozytenspender enthaltenen lgE-Antikörper sowie mit Graspollenallergen-spezifischen lgE aus Seren von anderen Graspollenallergikern eine deutlich verminderte Bindungsfähigkeit aufweisen.

Dieser Effekt, der weder bei den natürlich vorkommenden noch bei den rekombinanten Allergenen auftritt, ist deshalb wünschenswert, weil
- die IgE-vermittelten Nebenwirkungen bei der Hyposensibilisierung vermieden oder zumindest stark reduziert werden,
- die Erkennung der modifizierten rekombinanten Allergene durch die TH-Gedächtnislymphozyten der Allergiker gewährleistet ist,
- damit Voraussetzung für die Normalisierung der beim Allergiker gestörten Balance der unterschiedlich differenzierten TH-Subpopulationen gegeben ist,
- die therapeutische Wirkung durch Anergisierung und/oder Eliminierung der allergenreaktiven T-Zellen sowie die funktionelle Umorientierung von einer TH2-dominierten zu einer TH0/TH1-ausgerichteten spezifischen T-Zell-Population möglich wird,
- die Regulation der Immunglobulinsynthese von der für den Allergiker typischen Bildung spez. IgE-Antikörper (TH2-kontrolliert) zur bevorzugten Synthese von IgG-Antikörpern (TH1-kontrolliert) erfolgen kann,
- und dadurch bei einer Behandlung mit den erfindungsgemäßen modifizierten rekombinanten Allergene eine deutliche Verbesserung des Befindens der Patienten zu erwarten ist.

Vorzugsweise werden die mit dem erfindungsgemäßen Verfahren identifizierten modifizierten rekombinanten Allergene von den Hauptallergenen der Gruppe 5, aber auch von der Gruppe 1 abgeleitet. Insbesondere stammen die erfindungsgemäßen Allergene von dem Hauptallergen Phl p 5b ab.

Die Sequenz von Phl p 5b lautet gemäß dem Einbuchstabencode für Aminosäuren folgendermaßen :

ADAGYAPATPAAAGAAAGKATTEEQKLIEDINVGFKAAVAAAASVPAADK
1 10 20 30 40 50
FKTFEAAFTSSSKAAAAKAPGLVPKLDAAYSVAYKAAVGATPEAKFDSFV
51 60 70 80 90 100
ASLTEALRVIAGALEVHAVKPVTEEPGMAKIPAGELQIIDKIDAAFKVAA
101 110 120 130 140 150
TAAATAPADDKFTVFEAAFNKAIKESTGGAYDTYKCIPSLEAAVKQAYAA
151 160 170 180 190 200
TVAAAPQVKYAVFEAALTKAITAMSEVQKVSQPATGAATVAAGAATTAAG
201 210 220 230 240 250
AASGAATVAAGGYKV
251 260 265

Zur Ermittlung der DNA- bzw. Aminosäuresequenzen sind folgende Schritte notwendig:

Die allergenen Bestandteile der nach üblichen Verfahren hergestellten Extrakte werden identifiziert und ihre wesentlichen physikochemischen Parameter charakterisiert. Die Identifizierung als Allergen erfolgt durch Nachweis ihrer Bindungsfähigkeit an IgE-Antikörper von Allergikern. In der Regel benutzt man hierzu an sich bekannte Methoden, wie SDS-PAGE, Isoelektrofokussierung und anschließendes Western Blotting mit Seren von Allergikern, wobei die Entwicklung nur der bindenden Antikörper des IgE-Isotyps vorgenommen wird. Es ist dabei zu beachten, daß ausreichend viele Typen von klinisch gesicherten Allergikern (als Mindestanzahl ist hier ein Wert von 20 anzusetzen) verwendet werden. Alternativ können auch andere Methoden, wie z.B. die CIE oder CRIE eingesetzt werden.

Diese so identifizierten und charakterisierten Allergene aus Graminaenpollen können analytisch präpariert werden, so daß eine N-terminale Aminosäurebestimmung möglich ist. Weiterhin können die Allergene biochemisch gereinigt und zur Herstellung monoklonaler Antikörper verwendet werden. Diese monoklonalen Antikörper können, ebenso wie die IgE-Antikörper in den Seren von Allergikern, zur immunologischen Identifizierung und Charakterisierung der Allergene aus natürlichen Quellen oder der Moleküle, die durch die Rekombinantentechnik hergestellt werden, benutzt werden.

Ausgehend von diesen Informationen über Allergene und den Mitteln zur Identifizierung ist es möglich, die Allergene nach bekannten gentechnischen Verfahren zu klonieren und als rekombinante Allergene zu exprimieren. Die DNA-Klone der nach üblichen Verfahren gewonnenen und charakterisierten rekombinanten Allergene sind die Basis für die gentechnische Modifikation die zu den erfindungsgemäßen modifizierten, rekombinant hergestellten Allergenmolekülen führen.

Um die Reaktivität der erfindungsgemäßen modifizierten rekombinanten Allergene zu gewährleisten, ist außerdem die Identifizierung der T-Zell-Epitope erforderlich.

Grundlage hierfür ist die Kenntnis der Aminosäuresequenz der in Frage kommenden Allergene oder die entsprechend zugrundeliegende DNA-Sequenz. Die Aminosäuresequenz wird in der Regel aus der DNA-Sequenz der rekombinanten Allergene deduziert. Im Rahmen dieser Erfindung sind somit zu jeder angegebenen Peptidsequenz auch die zugehörigen DNA-Sequenzen mit eingeschlossen, auch wenn diese nicht explizit offenbart werden, da sie auf bekannte und einfache Weise aus den Peptidsequenzen herleitbar sind.

Basierend auf der Aminosäuresequenz wird eine Serie von überlappenden Oligopeptiden nach üblichen Verfahren, wie z.B. Festphasensynsthese nach modifizierten Merrifield-Techniken, hergestellt, wobei die gesamte Sequenz der Allergene abgedeckt wird. Geeignet sind hierbei Oligopeptide mit jeweils 6 - 20, vorzugsweise 9 -15 Aminosäureresten. Ganz besonders geeignet sind Dodecapeptide mit einem Versatz um jeweils 3 Aminosäuren, die überlappend die gesamte Sequenz des jeweiligen Allergens abdecken.

Zur Identifizierung der T-Zell-Epitope werden von GraminaenpollenAllergikern T-Zell-Klone durch wiederholte Stimulierung mit dem gereinigten natürlichen oder rekombinant hergestellten in Frage kommenden Allergen nach dem üblichen Verfahren etabliert (**Lit.**). Hierzu muß eine repräsentative Anzahl von T-Zell-Klonen, die von ausreichend vielen Spendern abstammen, etabliert werden.

Diese T-Zell-Klone werden mit den oben beschriebenen überlappenden Peptiden inkubiert und deren Fähigkeit, die T-Zellen zur Proliferation zu stimulieren, getestet. Die Proliferation wird durch Einbau von [³H]-Thymidin mit an sich üblichen Verfahren bestimmt. Diejenigen Oligopeptide, die eine ausreichende Proliferation der T-Zell-Klone auslösen, werden als Peptidliganden, die den T-Zell-Epitopen entsprechen, angesehen. Die so bestimmten T-Zell-Epitope dienen zur Festlegung von T-Zell-reaktiven Bereichen der Allergene, die ihrerseits die Basis für die Konstruktion der erfindungsgemäßen modifizierten rekombinanten Allergene darstellen.

Um die Reaktivität von modifizierten rekombinanten Allergenen mit den T-Lymphozyten, die bei Allergikern auftreten, zu gewährleisten, werden die T-Zell-reaktiven Bereiche, die die immundominanten T-Zell-Epitope einschließen, von Veränderungen hinsichtlich der Primärstruktur teilweise oder vollständig ausgeschlossen.

In den verbleibenden Bereichen der Polypeptide (Allergene) werden in den zugrundliegenden DNA-Sequenzen gentechnisch Mutationen vorgenommen, um eine veränderte Primärstruktur zu erzeugen. Durch diese veränderte Primärstruktur wird die Bindungsfähigkeit von sequenzabhängigen kontinuierlichen B-Zell-Epitopen zu den IgE-Antikörpern zerstört oder eingeschränkt und die Reaktivität von konformationsabhängigen, eventuell diskontinuierlichen Epitopen mit ihren Antikörpern durch die Ausbildung einer abgewandelten Tertiärstruktur als Folge der Primärmodifikation vollständig oder teilweise aufgehoben.

Die Mutationen können Substitutionen einzelner oder mehrerer Aminosäuren außerhalb der T-Zell-reaktiven Bereich darstellen. Solche Punktmutationen werden durch ortsspezifische Mutagenese mit Hilfe der Polymerase-Ketten-Reaktion (PCR) in die DNA, die z. B. für das rPhl p 5b kodiert, eingeführt. Als Matrize können dabei das Plasmid pGS13, ein Expressionsvektor (pMalc), der die cDNA für rPhl p 5b enthält, dienen. Zur PCR werden genspezifische Primer verwendet, die entsprechende Basenaustausche und gleichzeitig eine neue Restriktionsschnittstelle (Nhe I bzw. Sph I) enthalten. Die in der PCR amplifizierten Fragmente, die die Mutation tragen, wurden nacheinander in einen Klonierungsvektor ligiert, und dann das komplette Produkt in den pMalc-Expressionsvektor umkloniert.

Weiterhin können Mutationen durch unterschiedlich angeordnete Deletionen vorgenommen werden. Zur Herstellung der Deletionsmutanten werden in einer PCR mit Hilfe von genspezifischen Primern verkürzte 3'-terminale Fragmente der cDNA von rPHl p 5b hergestellt. Aus den Ausgangsvektoren (pGS12 oder pGS13) werden durch Restriktion an internen Schnittstellen größere 3'-terminale Fragmente entfernt, und an deren Stelle die jeweils kleineren in der PCR amplizierten Fragmente einligiert.

In analoger Art und Weise lassen sich Mutationen durch Additionen von ein oder mehreren Aminosäuren durch Einschub von zusätzlich DNA-Fragementen erzeugen.

Die gentechnisch mutierten DNA-Klone, die für modifizierte rekombinante Allergene kodieren, werden in geeignete Expressionsvektoren umkloniert und in geeigneten Wirtsorganismen zur Expression gebracht. Aus den Überständen oder Aufschlüssen dieser Wirtsorganismen werden in üblicher Weise die Fusionsproteine gereinigt und nach Abspaltung des Fusionsanteils die modifizierten rekombinanten Allergene mit üblichen biochemischen Methoden rein dargestellt. Es ist wichtig, das die modifizierten rekombinanten Allergene als reine Komponenten, die den natürlichen Allergenen entsprechen, für weitere Testmengen benutzt werden.

Die Auswirkungen der induzierten Mutationen auf die Allergenität, d.h. der Bindungsfähigkeit an IgE-Antikörper von Allergikern, der modifizierte rekombinante Allergene wird durch den EAST-Hemmtest qualitativ und quantitativ bestimmt. Dieser Assay zeigt, ob eine zu testende Substanz (modifiziertes rekombinantes Allergen) mit dem natürlichen Allergen und/oder dem rekombinanten Wildtyp identisch oder verschieden ist. Darüber hinaus läßt sich der Grad der immunchemischen Verwandschaft (Kreuzreaktivität) quantifizieren. Dieser EAST-Hemmtest berücksichtigt nur die Reaktion mit IgE-Antikörpern.

Als geeignete modifizierte rekombinante Allergene-Varianten werden diejenigen ausgewählt, die eine im Vergleich zum natürlichen Allergen und/oder rekombinanten Wildtyp mindestens um den Faktor 10² verringerte Hemmwirkung, gemessen als Pᵣₑₗ bei 50% Hemmung, aufweisen.

Die so ausgewählten modifizierte rekombinante Allergene-Varianten werden überprüft, ob die T-Zell-Reaktivität tatsächlich erhalten ist. Dazu wird in der ersten Phase ein Satz von T-Zellklonen, die mit Epitopen in den T-Zell-reaktiven Bereichen reagieren, zur Testung herangezogen.

Nur solche modifizierte rekombinante Allergene werden berücksichtigt, die die ausgewählten Klone zur Proliferation stimulieren.

In der zweiten Phase werden oligoklonale T-Zell-Linien, die durch mehrfache Stimulierung mit den betreffenden Allergen etabliert worden sind, zur Testung eingesetzt. Wiederum werden nur solche modifizierten rekombinanten Allergene berücksichtigt, die mindestens einen Stimulationsindex (SI) von 50 % des SI des Wildtyps bedingen.

In der dritten Phase werden polyklonale Kurzzeit-T-Zell-Kulturen aus dem peripheren Blut von Allergikern zur Testung eingesetzt.

Für die allergische Reaktion (Nebenwirkung) ist außer der Bindung des Allergens an das spez. lgE die allergeninduzierte, lgE-vermittelte Histaminfreisetzung durch allergische Effektorzellen von pathophysiologischer Bedeutung. Dabei ist auch die Reagibilität der Effektorzellen (Basophile, Mastzellen) und die Epitopspezifität der über FcεRl gebundenen lgE-Antikörper von Belang. Deshalb werden die modifizierte rekombinante Allergene-Varianten auf ihre Potenz zur Induktion der Histaminfreisetzung durch Degranulation IgE-beladener Basophiler, die aus dem Blut von Allergikern präpariert werden, getestet. Die modifizierte rekombinante Allergene-Varianten, die nach obigen Selektionsregime ausgewählt worden sind, müssen in diesem funktionellen Test eine starke reduzierte Fähigkeit zur Histaminfreisetzung aufweisen.

Die modifizierte rekombinante Allergene, die diese Anforderungen erfüllen, gewährleisten eine Reaktivität mit der Mehrheit der regulatorisch wirksamen TH-Zellen und besitzen aufgrund ihrer verminderten 1gE-Reaktivität die erforderlichen Eigenschaften, um als Therapeutika zur allergenspezifischen Immuntherapie (Hyposensibilisierung) von Graminaenpollenallergikem eingesetzt zu werden.

### Beispiel 1

### Identifizierung der T-Zell-Epitope zur Bestimmung der T-Zell-reaktiven Bereiche des Graspollenhauptallergens Phl p5

Für die Etablierung von T-Zell-Linien (TCL) und -Klonen (TCC), die mit dem Graspollenhauptallergen der Gruppe 5 des Lieschgrases (Phleum pratense) Phl p5 reagieren, wurden Patienten ausgewählt, die anamnestisch eine typische Symptomatik für eine Gräserpollenallergie (Rhinitis) angaben und einen positiven Hauttest (Pricktest) aufwiesen. Diese Patienten hatten zirkulierende spezifische IgE-Antikörper mit einer RAST-Klasse ≥ 3.

Es wurden je Patient 40 ml heparinisiertes Blut gewonnen. Danach wurden aus dieser Blutprobe nach üblichem Verfahren mittels Dichtegradientenzentrifugation periphere mononukleäre Zellen (PBMC) isoliert. Analoge Zellisolierungen erfolgten später, wenn die Gewinnung bestrahlter autologer Antigen-präsentierender Zellen (APZ) zur weiteren Charakterisierung der TCL und TCC notwendig war. Nach Zählung der PBMC wurden TCL mit Reaktivität auf Gruppe 5-Allergene in vitro wie folgt und bereits an anderen Stellen detailliert beschrieben (Lit. 1) etabliert: In 24 well - Mikrokulturplatten wurden pro Kavität 1,5 bis 2,0x10⁶ PBMC in 1 ml Kulturmedium (UltraCulture) 7 Tage lang unter Zugabe von immunaffinitätschromatographisch gereinigten natürlichen Phl p5-Allergenen (je 10 µg/well) stimuliert. Es wurden insgesamt 8 bis 10 dieser Kulturen angelegt. Die immunoaffinitätschromatographische Isolierung von Phl p 5 ist detailliert beschrieben (Lit. 2). Nach Ablauf der 7 Tage-Kultivierung wurde den Zellkulturen IL-2 (10 bis 20 IU/well) für weitere 5 bis 7 Tage zugegeben. Anschließend wurden alle Einzelkulturen gepoolt, über Dichtegradientenzentrifugation die T-Zellblasten angereichert und die gewonnene TCL im spezifischen Lymphozytenproliferationstest geprüft (siehe auch Lit. 1). Hierzu wurden in 96 well - Mikrokulturplatten im Dreifachansatz jeweils 2 x 10⁴/ml TCL-Blasten mit 5 x 10⁴/ml bestrahlten autologen APZ's kultiviert. Als spezifischer Antigenstimulus wurden 10-20 µg Phl p5-Allergen hinzugegeben. Nach 56 Stunden Inkubation wurde zu den Mikrokulturen ³H-markiertes Thymidin (1µCi/well) pipettiert. Weitere 16 Stunden später wurde die in den proliferierenden T-Zellblasten inkorporierte Radioaktivität in einem Beta-Counter (Matrix 96) gemessen. Die Resultate wurden als arithmetisches Mittel der Mehrfachansätze in Counts pro Minute (cpm) errechnet. Das Kriterium für die Qualität der TCL war der Stimulationsindex, der sich aus der Relation der cpm-Werte mit Phl p5-Zusatz zu denen ohne Phl p5-Zusatz ergab.

Nach Auswahl der TCL's wurden diese kloniert (s. Lit. 1). Dazu wurden 0,3 TCL-Blasten / well in einem Endvolumen von 0,2 ml in 96 well-Mikrokulturplatten (Rundboden) unter Zugabe bestrahlter allögener PBMC (5 x 10⁴/well), PHA (1,5 g/ml) und IL-2 (25 IU/ml) kultiviert. Nach 12 bis 14 Tagen wurden die Kulturen mit frischen bestrahlten PBMC, PHA und IL-2 gefüttert. Außerdem wurde alle 4 bis 5 Tage ein Mediumaustausch unter Zugabe von IL-2 (25 IU/ml) durchgeführt. Vor Durchführung des Phl p5 - spezifischen Proliferationstestes verstrich eine ca. 10 Tage lange Periode ohne Zugabe bestrahlter allogener PBMC. Die ausgewählten TCC wurden dann in 24 well - Mikrokulturplatten durch wiederholte Stimulation mit PHA, bestrahlten allogenen PBMC und IL-2 (50 IU/ml) vermehrt.

Nach Klonierung einer TCL (siehe unten) wurde die Spezifität der isolierten TCC wie eben beschrieben bestimmt. Für die TCC wurden Stimulationsindices von mindestens 5 als positiv gewertet. Auch die Bestimmung von T-Zellepitopen zur Festlegung der T-Zell-reaktiven Bereiche auf Gruppe 5 - Allergenen erfolgte mittels spezifischer Proliferationsteste, wobei hierfür jeweils 1-2 µg/ml synthetisierter Dodecapeptide eingesetzt wurden (siehe unten).

Für die Bestimmung der T-Zellepitope wurden insgesamt 86 überlappende synthetische Dodecapeptide verwendet, die auf der Grundlage der bekannten Primärstruktur des Phl p 5b-Allergens gemäß Bufe et al. (Lit. 3) hergestellt wurden. Die Herstellung dieser Peptide erfolgte mit einem kommerziellen Synthese-Kit der Firma CHIRON Mimotopes Peptide Systems / Clayton, Australien. Diese Peptide besaßen bezüglich ihrer Aminosäuresequenzen einen Überlappungsgrad von 9 Aminosäuren (Tab. 1). Die Reaktion von TCC auf eines der im spezifischen Proliferationstest verwendeten Peptide wurde als positiv bewertet, wenn der errechnete Stimulationsindex mindetens 5 betrug.

In die Untersuchungen wurden TCC von 18 Graspollenallergikern einbezogen. Von diesen konnten 54 T-Zellklone isoliert werden, die mit den Dodecapeptiden, basierend auf der Phl p 5b-Sequenz, spezifisch reagieren. Die Analyse dieser TCC zeigt eine deutliche Konzentration der Erkennung von Peptidliganden in 3 immundominanten T-Zell-reaktiven Bereichen. Von den 54 T-Zellklone reagieren 46, dies entspricht 85%, mit den Peptiden der 3 immundominanten T-Zell-reaktiven Bereichen A, B und C des Phl p 5b (Tab. 1a). Nur 8 T-Zellklone reagierten mit 5 anderen Peptidliganden, wobei 3 Peptide von jeweils 2 differenten Klonen erkannt werden. Der immundominante T-Zell-reaktive Bereich **A** umfaßt ein Peptid (27mer) entsprechend den Positionen 181-207, mit einer Kernregion bestehend aus den Aminosäuren 181-195. 28 der 54 Phl p 5b-reaktiven TCC, dies entspricht **51%**, reagieren allein mit diesem immundominanten Bereich **A**.

Mit den T-Zell-reaktiven Bereichen **C** (Position 16-48; 33mer) und **B** (Position 133-150) reagieren 9 (17%) bzw. 9 (17%) der T-Zell-Klone. Diese Konzentration der TH-Zellen des untersuchten Allergikerkollektivs auf die Erkennung von 3 immundominanten T-Zell-reaktiven Bereichen des Hauptallergens Phl p 5b läßt die Konstruktion von Phl p 5b-Mutanten zu, bei denen diese Bereiche von den Punkt-, Deletions- oder Additions-Mutationen nicht berührt werden. Damit ist die Voraussetzung gegeben, daß solche Allergen-Mutanten mit der bei Allergikern vorhandenen Allergen-reaktiven TH-Zellen spezifisch reagieren und diese im therapeutischen Sinne beeinflussen.

**Tab. 1: Auf der Phl p 5b-Sequenz basierende Dodecapeptide zur Bestimmung der T-Zell-reaktiven Bereiche**

| | | | |
|---|---|---|---|
| 1 | ADAGYAPATPAA | 44 | KIPAGELQIIDK |
| 2 | GYAPATPAAAGA | 45 | AGELQIIDKIDA |
| 3 | PATPAAAGAAAG | 46 | LQIIDKIDAAFK |
| 4 | PAAAGAAAGKAT | 47 | IDKIDAAFKVAA |
| 5 | AGAAAGKATTEE | 48 | IDAAFKVAATAA |
| 6 | AAGKATTEEQKL | 49 | AFKVAATAAATA |
| 7 | KATTEEQKLIED | 50 | VAATAAATAPAD |
| 8 | TEEQKLIEDINV | 51 | TAAATAPADDKF |
| 9 | QKLIEDINVGFK | 52 | ATAPADDKFTVF |
| 10 | IEDINVGFKAAV | 53 | PADDKFTVFEAA |
| 11 | INVGFKAAVAAA | 54 | DKFTVFEAAFNK |
| 12 | GFKAAVAAAASV | 55 | TVFEAAFNKAIK |
| 13 | AAVAAAASVPAA | 56 | EAAFNKAIKEST |
| 14 | AAAASVPAADKF | 57 | FNKAIKESTGGA |
| 15 | ASVPAADKFKTF | 58 | AIKESTGGAYDT |
| 16 | PAADKFKTFEAA | 59 | ESTGGAYDTYKC |
| 17 | DKFKTFEAAFTS | 60 | GGAYDTYKCIPS |
| 18 | KTFEAAFTSSSK | 61 | YDTYKCIPSLEA |
| 19 | EAAFTSSSKAAA | 62 | YKCIPSLEAAVK |
| 20 | FTSSSKAAAAKA | 63 | IPSLEAAVKQAY |
| 21 | SSKAAAAKAPGL | 64 | LEAAVKQAYAAT |
| 22 | AAAAKAPGLVPK | 65 | AVKQYAATYAA |
| 23 | AKAPGLVPKLDA | 66 | QAYAATVAAAPQ |
| 24 | PGLVPKLDAAYS | 67 | AATVAAAPQVKY |
| 25 | VPKLDAAYSVAY | 68 | VAAAPQVKYAVF |
| 26 | LDAAYSVAYKAA | 69 | APQVKYAVFEAA |
| 27 | AYSVAYKAAVGA | 70 | VKYAVFEAALTK |
| 28 | VAYKAAVGATPE | 71 | AVFEAALTKAIT |
| 29 | KAAVGATPEAKF | 72 | EAALTKAITAMS |
| 30 | VGATPEAKFDSF | 73 | LTKAITAMSEVQ |
| 31 | TPEAKFDSFVAS | 74 | AITAMSEVQKVS |
| 32 | AKFDSFVASLTE | 75 | AMSEVQKVSQPA |
| 33 | DSFVASLTEALR | 76 | EVQKVSQPATGA |
| 34 | VASLTEALRVIA | 77 | KVSQPATGAATV |
| 35 | LTEALRVIAGAL | 78 | QPATGAATVAAG |
| 36 | ALRVIAGALEVH | 79 | TGAATVAAGAAT |
| 37 | VIAGALEVHAVK | 80 | ATVAAGAATTAA |
| 38 | GALEVHAVKPVT | 81 | AAGAATTAAGAA |
| 39 | EVHAVKPVTEEP | 82 | AATTAAGAASGA |
| 40 | AVKPVTEEPGMA | 83 | TAAGAASGAATV |
| 41 | PVTEEPGMAKIP | 84 | GAASGAATVAAG |
| 42 | EEPGMAKIPAGE | 85 | SGAATVAAGGYK |
| 43 | GMAKIPAGELQI | 86 | GAATVAAGGYKV |

**Tab. 1a: Kartierung der T-Zell-reaktiven Bereiche des Graspollenhauptallergens Phl p 5**

| **TCC** | **Stimulierende Peptidliganden (12mer)** | **Immundominanter T-Zellreaktiver Bereich** | | | **Minor Epitop** |
|---|---|---|---|---|---|
| | | **A** | **B** | **C** | |
| DW 8 | 139-150 | | + | | |
| DW 14 | 196-207 | + | | | |
| DW 16 | 181-192, 184-195 | + | | | |
| DW 23 | 181-192 | + | | | |
| DW 25 | 181-192, 184-195 | + | | | |
| DW 28 | 184-195 | + | | | |
| CBH 1 | 211-222, 214-225 | | | | + |
| CBH 10 | 211-222 | | | | + |
| JR 6a | 22-33, 25-36 | | | + | |
| JR 6b | 136-147, 139-150 | | + | | |
| JR 7a | 28-39, 31-42 | | | + | |
| JR 7b | 136-147, 139-150 | | + | | |
| JR 9 | 181-192, 184-195 | + | | | |
| JR 10 | 19-30 | | | + | |
| JR 11 | 49-60 | | | | + |
| JR 13 | 181-192, 184-195 | + | | | |
| JR 15 | 181-192, 184-195 | + | | | |
| JR 19a | 31-42 | | | + | |
| JR 19b | 136-147 | | + | | |
| JR 24 | 97-108, 100-111 | | | | + |
| JR 25 | 181-192, 184-195 | + | | | |
| JR 27 | 184-195 | + | | | |
| KS 1 | 181-192, 194-195 | + | | | |
| KS 2 | 181-192, 194-195 | + | | | |
| KS 3 | 181-192, 194.195 | + | | | |
| KS 4 | 181-192, 194-195 | + | | | |
| KS 5 | 181-192, 194.195 | + | | | |
| KSE 18 | 43-54 | | | | + |
| UD 6 | 112-123 | | | | + |
| GE 4 | 136-147, 139-150 | | + | | |
| GE 7 | 136-147 | | + | | |
| GE 12 | 37-48 | | | + | |
| AS 4 | 181-192, 184-195 | + | | | |
| AS 5 | 181-192, 184-195 | + | | | |
| UZH 2 | 136-147, 139-15 | | + | | |
| UZ 25 | 97-108 | | | | + |
| CB 1 | 190-201, 193-204 | + | | | |
| CB 2 | 181-192, 184-195 | + | | | |
| CB 7 | 25-36 | | | + | |
| CB 10 | 181-192, 184-195 | + | | | |
| CB 14 | 181-192 | + | | | |
| MF 11 | 184-195 | + | | | |
| AH 19 | 16-27 | | | + | |
| AH 26 | 139-150 | | + | | |
| JMD 3 | 133-144 | | + | | |
| 45 | | A22 | 9B | 7c | 7 |
| II 3.2A 12 | 31-42 | | | + | |
| II 12.7F11 | 196-207 | + | | | |
| II 12.5C10 | 187-198 | + | | | |
| II 17.9E5 | 184-195 | + | | | |
| II 17.1D8 | 184-195 | + | | | |
| II 17.11C2 | 184-195 | + | | | |
| II 17.19A1 | 193-204 | + | | | |
| II 17.12F5 | 25-36 | | | + | |
| II 17.3C10 | 49-60, 52-63 | | | | + |
| 54 | | 28 | 9 | 9 | 8 |

### Literatur:

1. Müller WD. Karamfilov T. Fahlbusch B, Vogelsang H, Jäger L: "Analysis of human T cell clones reactive with group V grass pllen allergens". Int. Arch. Allergy Immunol. 1994, 105:391-396.
2. Jung K, Fahlbusch B, Müller WD, Hermann D, Diener C, Jäger L: "Isolation of timothy (Phleum pratense) allergens using affinity chromatography with monoclonal antibodies". Allergy Immunol (Leipzig) 1989, 35:287-294
3. Bufe A, Schramm G, Keown MB, Schlaak M, Becker WM: "Major allergen Phl p 5b in timothy grass is an novel pollen Rnase". FEBS Letters 1995, 263:6-12.

### Beispiel 2

### Herstellung der Punktmutanten PM1, PM2 (D⁴⁸ → L, K⁵⁰ → A) und PM3 (A¹³ → C) des rPhl p 5b

### PM2:

Als Ausgangsvektor diente das Plasmid pGS13. Es handelt sich hierbei umd en pMalc-Vektor (Biolabs), der die cDNA für das wt rPhl p 5b Bam HI / Hind III kloniert enthält. In einer PCR-Reaktion wurden die Fragmente 1 (Bp: 1 - 153) und 2 (Bp: 141 - 1374) der cDNA des rPhl p 5b amplifiziert. Dazu wurden folgende Primer verwendet (Restriktionsschnittstellen sind unterstrichen):
Fragment 1:
   Phl p 5b sense:
      5'-ATATGGATCCATCGAGGGAAGGGCCGATGCCGGCTACGCC-3
   MP1 antisense:
      5'-GAACGCTAGCGCCGCAGGGACGCTGGC-3'
Fragment 2:
   MP1 sense:
      5'-GCGCTAGCGTTCAAGACCTTCGAG-3'
   Phl p 5b antisense:
      5'-ATATAAGCTTTCCTCTGAAGGAAGGCAACCC-3'

Die beiden Mutagenese-Primer MP1 sense und MP1 antisense enthalten 6 Basenaustausche gegenüber der wt-Sequenz, die zusätzlich eine neue Restriktionsschnittstelle für das Enzym Nhe1 1, ergeben.

Das amplifizierte Fragment 1 wurde Bam Hl /Nhe I verdaut und in den Vektor pUH89 (Jekel et al., Gene: 154, 55-59; 1995) kloniert. Das resultierende Plasmid pGS10 wurde erneut mit Nhe I / Hind III restringiert und in diese Schnittstellen das Fragment 2 (Nhe I / Hind III) eingebaut. Dieses Plasmid pGS11 enthält die komplette cDNA, die für das rPhl p 5b kodiert, mit den gewünschten Basenaustauschen. Zur Expression der Punktmutante rPhl p 5b PM2 wurde die mutierte cDNA in die Bam HI / Hind III-Schnittstellen in den Expressionsvektor pMalc umkloniert. Das entstandene Plasmid wurde mit pGS21 bezeichnet.

Die Punktmutante rPhl p 5b PM1 wurde analog zu PM2 hergestellt. Sie enthält, bedingt durch einen PCR-Fehler, eine zusätzliche Punktmutation: N³² → D.
Zur Klonierung dieser Punktmutante wurde die gesamte cDNA von rPhl p 5b im Vektor p GS13 in einer PCR mit folgenden Primern appliziert.

PCysM1:
5'ATATGGATCCATCGAGGGTAGGGCCGATGCCGGCTACGCCCCGGC CACCCCGGCTGCATGCGGAGCG-3'

Phl p 5b antisense: siehe oben.

Der Mutagenese-Primer PCysM1 enthält 3 Basenaustausche gegenüber der wt-Sequenz, die zum Austausch eines Alanin-Restes gegen einen Cystein-Rest führen, und gleichzeitig eine neue Restriktionsschnittstelle für das Enzym Sph I ergeben. Das PCR-Produkt wurde direkt in den Expressionsvektor pMalc (Bam HI / Hind III) kloniert. Der resultierende Vektor wurde mit pCysM1 bezeichnet. Die erfolgreiche Mutagenese wurde in einer Restriktionsanalyse mit Sph I überprüft.

### Beispiel 3

### Herstellung der Deletionsmutanten DM1 (ΔK⁵⁰ - P¹³², D⁴⁹ → L), DM2 (ΔF⁵¹ - G¹⁷⁸, D⁴⁹ → L, K⁵⁰ → A) und DM3 (ΔA¹⁵⁴ - T¹⁷⁷, A²²⁰ → T)

Als Ausgangsvektor für die Klonierung der Deletionsmutante DM1 diente das Plasmid pGS21 (siehe oben). In einer PCR wurde das Fragment Bp 399 - 1374 der cDNA von rPhl p 5b amplifiziert unter Verwendung der folgenden Primer:
MP2 sense:
   5'-GCTAGCCGGCGAGCTGCAGATCATCG-3'

Phl p 5b antisense: siehe oben.

Der Vektor pGS21 wurde Nhe 1 / Bam Hl restringiert, vom herausgeschnittenen Fragment getrennt und in den Restvektor das ebenfalls Nhe I / Bam HI restringierte PCR-Produkt einligiert. Der daraus resultierende Vektor pDM1 enthält die cDNA des rPhl p 5b mit einer Deletion von 252 bp, die für die Deletionsmutante rPhl p 5bDM1 kodiert.
Die Deletionsmutanten DM2 und DM3 wurden analog hergestellt.

### Beispiel 4

### Nachweis der verminderten Allergenität (IgE-Reaktivität) der rekombinanten Phl p 5b-Mutanten durch den EAST-Hemmtest

Die Bindung der Allergene durch die lgE-Antikörper ist die Grundvoraussetzung für die Allergen-spezifische Aktivierung der Effektorzellen (Mastzellen, Basophile u.a.) bei der Typ l-Allergie. Die Bindung der Allergene an IgE-Antikörper kann am besten mit der Allergenspezifischen Hemmung des Enzym-Allergen-Sorbent-Tests (EAST) qualitativ und quantitativ erfaßt werden. Der EAST-Hemmtest wird wie folgt ausgeführt. Mikrotiterplatten werden mit Allergen (natürliches oder rekombinantes Phl p 5 bzw. Phl p 5b) beschichtet (1 µg/ml). Nach Entfernung der nicht gebundenen Allergenmoleküle durch Waschung werden unspezifische Plastbindungsstellen mit Rinderserumalbumin (0,5%) blockiert. Anti-IgE von Allergikern, als repräsentativer Pool von 10-30 Spendern oder als Einzelserum, wird in einer geeigneten Verdünnung mit den Allergen-beschichteten Mikrotiterplatten inkubiert. Die gebundenen allergenspezifischen lgE-Antikörper werden mittels Enzym-gekoppel-tem Anti-IgE (z.B. Alk. Phosphatase - a-IgE) quantifiziert. Diese Bindung wird durch lösliches Allergen oder die zu prüfende Substanz (Allergen-Mutanten) in Abhängigkeit von der Konzentration gehemmt. Als Bezug dient die Hemmkurve mit dem gereinigten natürlichen Allergen Phl p 5b.

Mit dem repräsentativen Allergikerserum-Pool Bor 18/100 (20 Spender) ergeben sich die in Abb. 1 dargestellten Hemmkurven.

Das rPhl p 5b (Wildtyp) und die PM3 zeigen dem affinitätschromatographisch gereinigten, natürlichen Phl p 5b ähnliche Bindungskurven. Geringfügige Unterschiede werden durch bessere Hemmwirkung im niedrigeren Bereich sowie durch schlechtere Hemmung in hohen Konzentrationen sichtbar. Die Ursache hierfür ist unbekannt, könnte aber in geringfügig differierenden Konformationsepitopen begründet sein.

Die Punktmutante PM1 zeigt diesen Effekt im höheren Bereich etwas stärker ausgeprägt. Deutlich verminderte Hemmwirkung weisen die Deletionsmutanten DM1 und DM3 auf. Dies belegt die stark reduzierte Allergenität dieser Allergen-Mutanten, die damit mit chemisch modifizierten Allergen (Allergoiden) vergleichbar sind.

Die Deletionsmutanten DM2 und DM2* zeigen eine extrem geringe Hemmwirkung der Allergen-IgE-Reaktion. Dies zeigt, daß die Allergenität dieser Mutanten weitgehend elimiert ist. Ein anderer Allergiker-Serumpool (We 6/97) sowie die Einzelseren der Allergiker ll3, ll12 und ll17 zeigen zwar leichte Variationen der Hemmkurven mit den Mutanten, bestätigen aber, daß die Deletionsmutanten DM1 und DM3 eine stark reduzierte Allergenität aufweisen (Abb. 2-5). Die Hemmwirkung der Deletionsmutanten DM2 und DM2* ist auf eine geringe Restaktivität eliminiert. Die Punktmutationen PM1 und PM3 zeigen keine oder nur eine meist geringe Reduktion der Allergenität (z.B. PM1 mit Pool We 6/97 und Einzelserum II 17). Die Hemmkapazität der modifizierten Allergene kann durch Berechnung der Prel-Werte bei 25%iger oder 50%iger Hemmung quantifiziert werden (1). Die entsprechenden Hemmwerte und sowie die Allergene Potenz (Prel) gemessen bei 25 bzw. 50% Hemmung sind in den Tabellen 2-6 für die Serumpools und Einzelseren dargestellt.

Die Deletionsmutation DM2 und DM2* zeigen den Verlust der Allergenität durch die extrem kleinen oder nicht mehr sinnvoll bestimmbaren Prel-Werte. Die Punktmutationen PM1 und PM3 zeigen teilweise einen Allergenitätsverlust, der aber für praktische Anwendung nicht ausreicht.
Die Deletionsmutanten DM 1 und DM 3 weisen eine starke Reduzierung der Allergenität auf. Die Reduzierung der IgE-Reaktivität ist besser oder vergleichbar mit den bisher bekannten chemisch modifizierten Allergenen und macht dadurch diese Mutanten zu besonders geeigneten Kandidaten für die Immuntherapie.

### Literatur

Anderson MC and Baer H: Methology for RAST inhibition. Food and Drug Administration, Bethesda, Maryland, U.S.A. (1986).

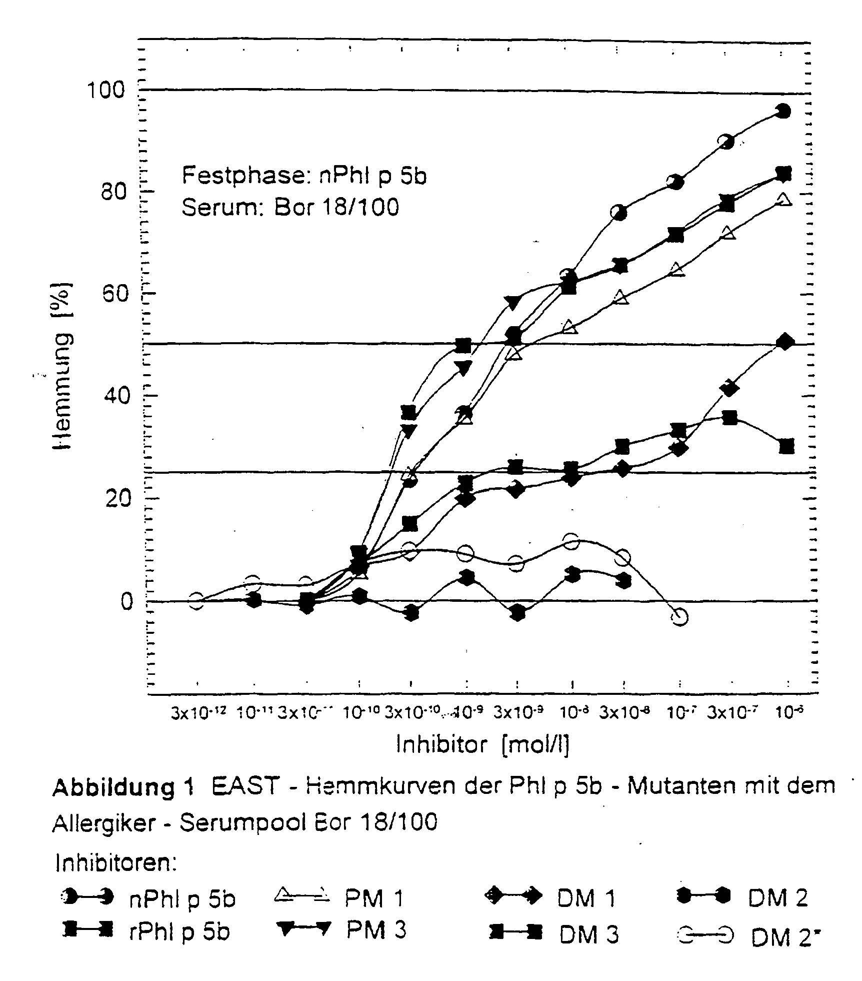
Festphase: nPhl p 5b
Serum: Bor 18/100
100
80
60
40
20
0
Hemmung [%]
3x10-12 10-11 3x10-11 10-10 3x10-10 10-9 3x10-9 10-8 3x10-8 10-7 3x10-7 10-6
Inhibitor [mol/l]
Abbildung 1 EAST - Hemmkurven der Phl p 5b - Mutanten mit dem
Allergiker - Serumpool Bor 18/100
Inhibitoren:
nPhl p 5b PM 1 DM 1 DM 2
rPhl p 5b PM 3 DM 3 DM 2*

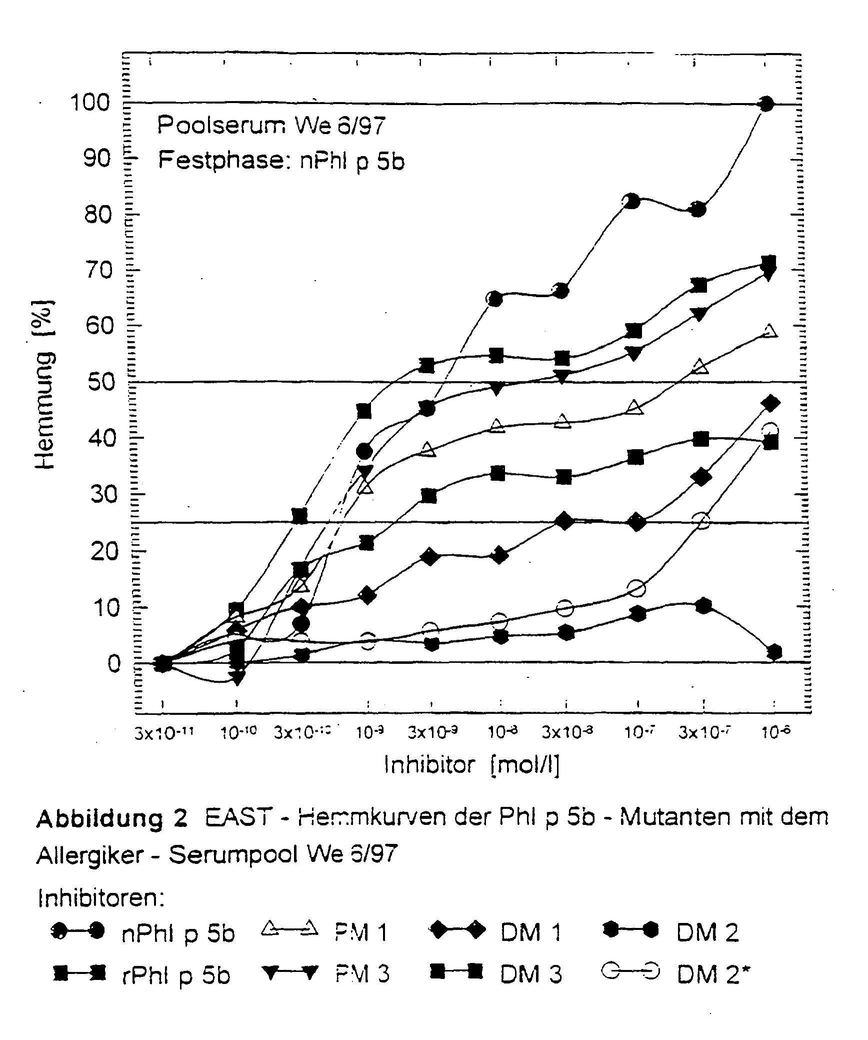

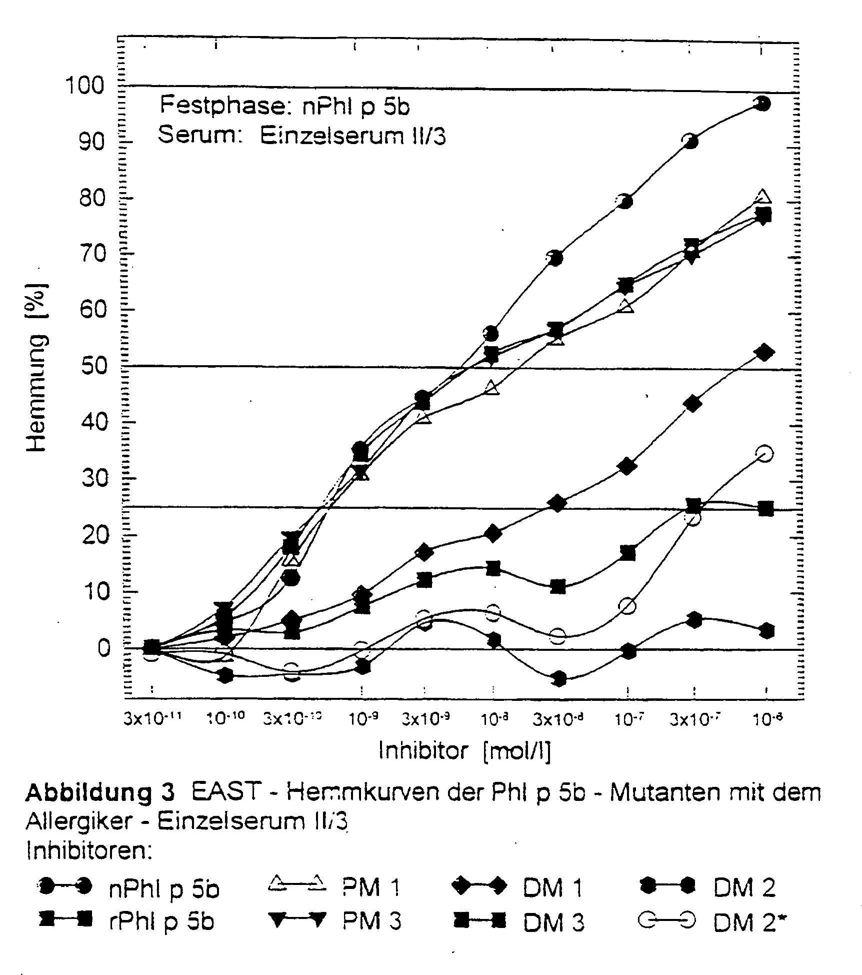

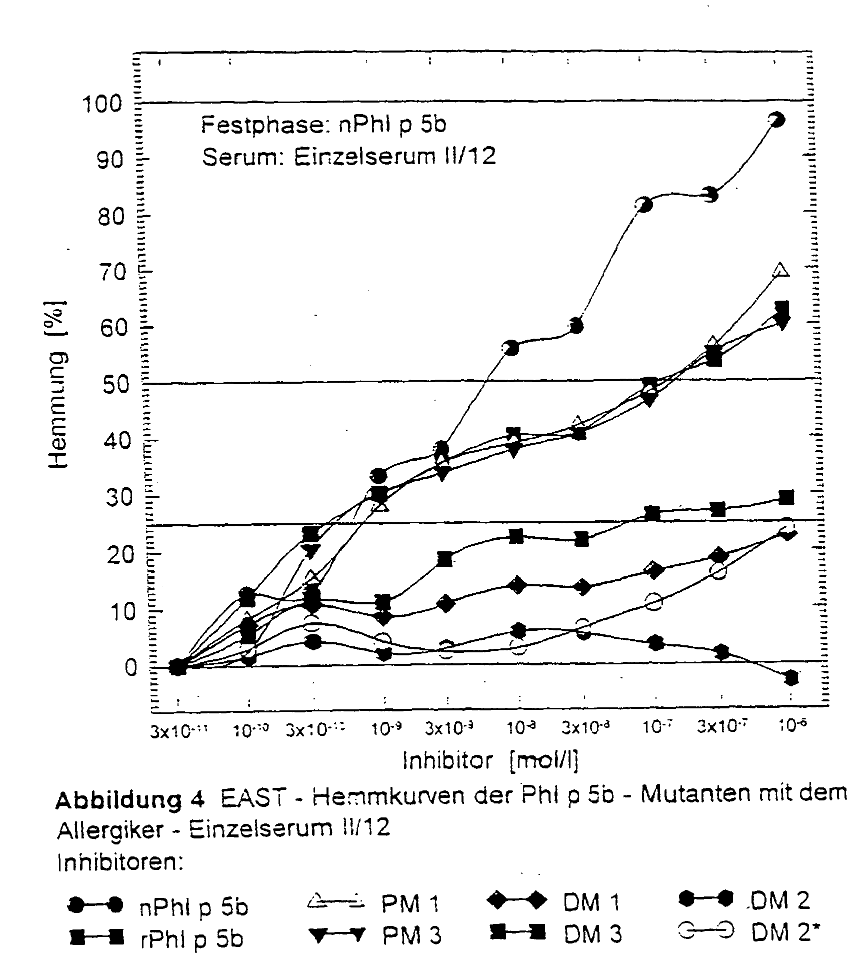

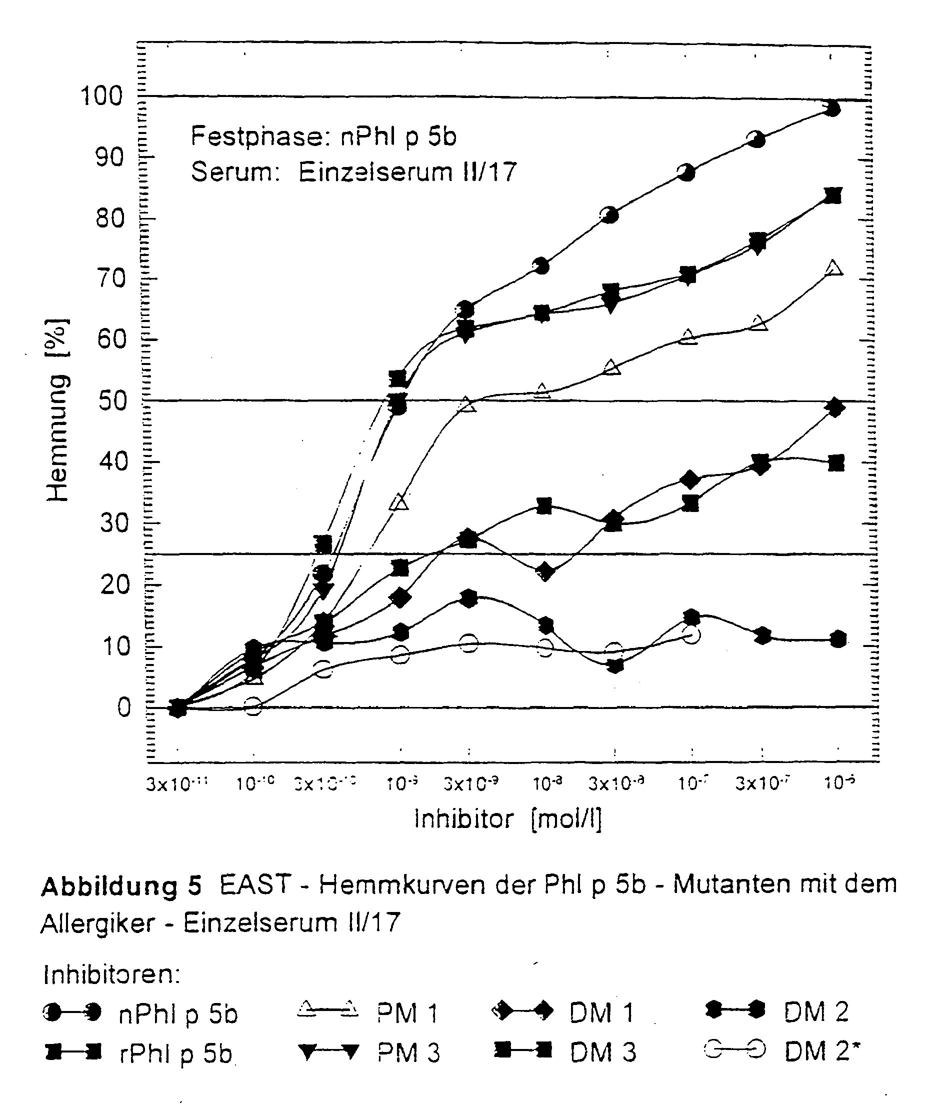

**Tabelle 2: Allergene Potenz (Pᵣₑₗ.) der rekombinanten Phl p 5b-Mutanten im Vergleich zu rekombinantem und nativem Phl p 5b mit dem Allergiker-Serumpool Bor 18/100**

| Inhibitor | Hemmwert¹ [mol/l] | | Allergene Potenz(Pᵣₑₗ)² | |
|---|---|---|---|---|
| | 25% | 50% | 25% | 50% |
| n Phl p 5b | 3,3 × 10⁻¹⁰ | 4,2 × 10⁻⁹ | 1,000 | 1,000 |
| r Phl p 5b | 2,0 × 10⁻¹⁰ | 5,0 × 10⁻⁹ | 1,709 | 0,8410 |
| PM1 | 4,5 × 10⁻¹⁰ | 1,2 × 10⁻⁸ | 0,739 | 0,3490 |
| PM3 | 2,0 × 10⁻¹⁰ | 4,8 × 10⁻⁹ | 1,641 | 0,8640 |
| DM1 | 8,6 × 10⁻⁹ | 2,8 × 10⁻⁸ | 0,039 | 0,0015 |
| DM2 | 8,3 × 10¹³ | 2,3 × 10³⁸ | 4,0 × 10⁻²³ | 1,8 × 10⁻⁴⁵ |
| DM3 | 1,2 × 10⁻⁸ | 4,1 × 10⁻⁵ | 0,028 | 0,0001 |
| DM2* | 5,0 × 10²³ | 2,3 × 10⁶⁶ | 6,7 × 10⁻³⁴ | 2,0 × 10⁻⁷⁵ |

| | | | | |
|---|---|---|---|---|
| ¹Hemmwerte: Konzentrationen d. Inhibitoren bei 25% bzw. 50% Hemmung | | | | |
| ²Allergene Potenz: relativ zu nativem Phlp5b bei 25% bzw. 50% Hemmung | | | | |

**Tabelle 3: Allergene Potenz (Pᵣₑₗ.) der rekombinanten Phl p 5b-Mutanten im Vergleich zu rekombinantem und nativem Phl p 5b mit dem Allergiker-Serumpool WE 6/97**

| Inhibitor | Hemmwert¹ [mol/l] | | Allergene Potenz(Pᵣₑₗ)² | |
|---|---|---|---|---|
| | 25 % | 50 % | 25 % | 50 % |
| n Phl p 5b | 5,1 × 10⁻¹⁰ | 6,1 × 10⁻⁹ | 1,000 | 1,000 |
| r Phl p 5b | 3,0 × 10⁻¹⁰ | 1,4 × 10⁻⁸ | 1,697 | 0,4400 |
| PM1 | 1,2 × 10⁻⁹ | 1,2 × 10⁻⁷ | 0,415 | 0,0510 |
| PM3 | 8,3 × 10⁻¹⁰ | 3,0 × 10⁻⁸ | 0,611 | 0,2030 |
| DM1 | 2,3 × 10⁻⁸ | 1,7 × 10⁻⁵ | 0,022 | 0,0004 |
| DM2 | 1,9 × 10⁸ | 2,7 × 10²¹ | 2,6 × 10⁻¹⁵ | 2,3 × 10⁻³⁰ |
| DM3 | 5,1 × 10⁻⁹ | 2,9 × 10⁻⁸ | 0,099 | 0,0020 |
| DM2* | 4,6 × 10⁻⁷ | 1,5 × 10⁻³ | 0,001 | 4,0 × 10⁻⁸ |

| | | | | |
|---|---|---|---|---|
| ¹Hemmwerte: Konzentrationen d. Inhibitoren bei 25% bzw. 50% Hemmung | | | | |
| ²Allergene Potenz: relativ zu nativem Phlp5b bei 25% bzw. 50% Hemmung | | | | |

**Tabelle 4: Allergene Potenz (Pᵣₑₗ.) der rekombinanten Phl p 5b-Mutanten im Vergleich zu rekombinantem und nativem Phl p 5b mit dem Allergiker-Einzelserum II/3**

| Inhibitor | Hemmwert¹ [mol/l] | | Allergene Potenz(Pᵣₑₗ)² | |
|---|---|---|---|---|
| | 25% | 50% | 25% | 50% |
| n Phl p 5b | 5,1 × 10⁻¹⁰ | 5,9 × 10⁻⁹ | 1,000 | 1,000 |
| r Phl p 5b | 5,6 × 10⁻¹⁰ | 1,4 × 10⁻⁸ | 0,9030 | 0,4190 |
| PM1 | 8,6 × 10⁻¹⁰ | 1,9 × 10⁻⁸ | 0,5950 | 0,3140 |
| PM3 | 5,5 × 10⁻¹⁰ | 1,5 × 10⁻⁸ | 0,9220 | 0,3990 |
| DM1 | 1,2 × 10⁻⁸ | 1,7 x 10⁻⁸ | 0,0420 | 0,0035 |
| DM2 | 6,6 × 10¹⁰ | 5,2 × 10²⁷ | 7,7 × 10⁻²⁰ | 1,1 × 10⁻³⁸ |
| DM3 | 1,1 × 10⁻⁶ | 0,032 | 0,0004 | 1,8 × 10⁻⁷ |
| DM2^{*} | 2,1 × 10⁻⁶ | 0,010 | 0,0002 | 5,9 × 10⁻⁷ |

| | | | | |
|---|---|---|---|---|
| ¹Hemmwerte: Konzentrationen d. Inhibitoren bei 25% bzw. 50% Hemmung | | | | |
| ²Allergene Potenz: relativ zu nativem Phip5b bei 25% bzw. 50% Hemmung | | | | |

**Tabelle 5: Allergene Potenz (Pᵣₑₗ.) der rekombinanten Phl p 5b-Mutanten im Vergleich zu rekombinantem und nativem Phl p 5b mit dem Allergiker-Einzelserum III12**

| Inhibitor | Hemmwert¹ [mol/l] | | Allergene Potenz(Pᵣₑₗ)² | |
|---|---|---|---|---|
| | 25 % | 50 % | 25 % | 50 % |
| n Phl p 5b | 5,2 × 10⁻¹⁰ | 6,8 × 10⁻⁹ | 1,000 | 1,000 |
| r Phl p 5b | 8,7 × 10⁻¹⁰ | 7,3 × 10⁻⁸ | 0,597 | 0,093 |
| PM1 | 1,3 × 10⁻⁹ | 8,3 × 10⁻⁸ | 0.391 | 0,082 |
| PM3 | 1,3 × 10⁻⁹ | 9,1 × 10⁻⁸ | 0,389 | 0,075 |
| DM1 | 1,5 × 10⁻⁵ | 68,0 | 3,4 × 10⁻⁵ | 1,0 × 10⁻¹⁰ |
| DM2 | 3, 8 × 10¹⁰ | 4,4 × 10³⁰ | 1,4 × 10⁻¹⁹ | 1,6 × 10⁻³⁹ |
| DM3 | 4,5 × 10⁻⁸ | 0,0001 | 0,012 | 5,7 × 10⁻⁵ |
| DM2* | 196,0 | 7,4 × 10¹⁴ | 2,6 × 10⁻¹² | 9,2 × 10⁻²⁵ |

| | | | | |
|---|---|---|---|---|
| ¹ Hemmwerte: Konzentrationen d. Inhibitoren bei 25% bzw. 50% Hemmung | | | | |
| ²Allergene Potenz: relativ zu nativem Phip5b bei 25% bzw. 50% Hemmung | | | | |

**Tabelle 6: Allergene Potenz (Pᵣₑₗ.) der rekombinanten Phl p 5b-Mutanten im Vergleich zu rekombinantem und nativem Phl p 5b mit dem Allergiker-Einzelserum II/17**

| Inhibitor | Hemmwert¹ [mol/l] | | Allergene Potenz(Pᵣₑₗ)² | |
|---|---|---|---|---|
| | 25 % | 50 % | 25 % | 50 % |
| n Phl p 5b | 2,2 × 10⁻¹⁰ | 2,6 × 10⁻⁹ | 1,000 | 1,000 |
| r Phl p 5b | 2,1 × 10⁻¹⁰ | 4,7 × 10⁻⁹ | 1,045 | 0,5450 |
| PM1 | 6,4 × 10⁻¹⁰ | 2,2 × 10⁻⁸ | 0,336 | 0,1190 |
| PM3 | 2,5 × 10⁻¹⁰ | 5,5 × 10⁻⁹ | 0,855 | 0,4680 |
| DM1 | 6,5 × 10⁻⁹ | 2,0 × 10⁻⁸ | 0,033 | 0,0010 |
| DM2 | 73,9 | 6,4 × 10¹⁹ | 2,9 × 10⁻¹² | 4,1 × 10⁻²⁹ |
| DM3 | 5,6 × 10⁻⁹ | 5,0 × 10⁻⁸ | 0,038 | 0,0005 |
| DM2* | 0,0004 | 11675,0 | 5,3 × 10⁻⁷ | 2,2 × 10⁻¹³ |

| | | | | |
|---|---|---|---|---|
| ¹Hemmwerte: Konzentrationen d. Inhibitoren bei 25% bzw. 50% Hemmung | | | | |
| ²Allergene Potenz: relativ zu nativem Phip5b bei 25% bzw. 50% Hemmung | | | | |

### Beispiel 5

### Reduzierte Histaminfreisetzung von Basophilen durch die rPhl p 5b-Mutanten

Die hergestellte Punktmutante PM3 und die Deletionsmutanten DM1, DM2, DM2* und DM3 wurden auf ihre Fähigkeit, Histamin aus Basophilen freizusetzen, überprüft und mit dem Wildtyp rPhl p 5b verglichen.

Vor dem Histaminfreisetzungstest wurden zunächst die basophilen Leukozyten aus dem EDTA-Blut eines Allergikers (PS-W) über eine Dextran-Sedimentation angereichert und dann auf eine Endkonzentration von 100.000 Basophilen/ml eingestellt. Zur Freisetzung von Histamin aus den Basophilen wurden jeweils 200 µl der Zellsuspension mit 50 µl Antigenlösung 40 min. bei 37°C inkubiert. Dazu wurde das rPhl p 5b und die Mutanten in verschiedenen Konzentrationen eingesetzt (von 10⁻⁵ -10⁻¹² M). Das freigesetzte Histamin wurde in den jeweiligen Überständen mit Hilfes des Methyl-Histamin-RIA der Fa. Pharmacia nach der Vorschrift des Herstellers bestimmt.

Alle untersuchten rekombinanten Proteine beschrieben im Histaminfreisetzungstest mit zunehmender Konzentration die typische Glockenkurve (Abb. 6). Im Vergleich zum Wildtyp rPhl p 5b zeigte die Punktmutante keine signifikanten Unterschiede bezüglich ihrer Fähigkeit, Histamin freizusetzen. Für die Deletionsmutanten DM3, DM1 bzw. DM2 ergab sich, bei Bezug auf die Konzentraton, die eine 30%ige Histaminfreisetzung bewirkt, eine Reduktion um das 3-, 20-, bzw. 500-fache. Die Deletionsmutanten zeigen also eindeutig ein vermindertes Vermögen, Histamin aus Basophilen freizusetzen.

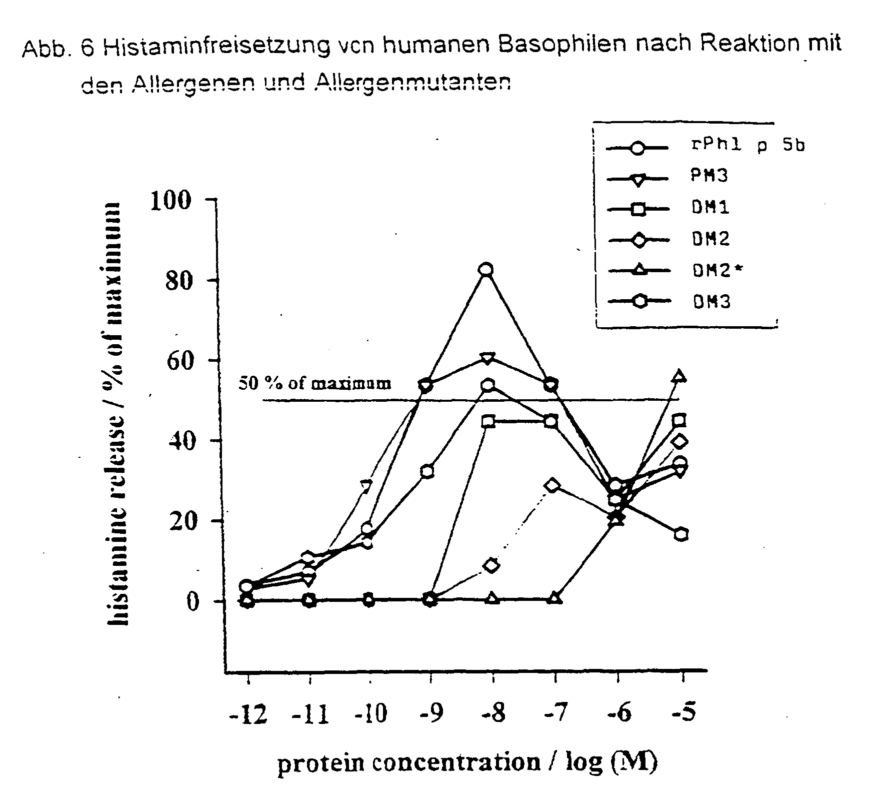
Abb. 6 Histaminfreisetzung vcn humanen Basophilen nach Reaktion mit
den Allergenen und Allergenmutanten


rPhl p 5b
PM3
DM1
DM2
DM2*
DM3

100
80
60
40
20
0

histamine release / % of maximum

50 % of maximum

-12 -11 -10 -9 -8 -7 -6 -5

protein concentration / log (M)

### Beispiel 6

### Nachweis der Reaktivität von rekombinanten Phl p 5b-Mutanten mit T-Zell-Klonen von Graspollenallergikern

Die Reaktivität der rekombinanten Phl p 5b-Mutanten wurde an etablierten T-Zell-Klonen (TCC) mit bekannter Spezifität getestet. Die TCC stammen von Graspollen-Allergikern (s. Bsp. 1) und sind gegen die T-Zell-reaktiven Bereiche A (Abb. 7), B (Abc. 8) und C (Abb. 9) gerichtet. Die T-Zellreaktivität wurde durch die Stimulierung der Klone zur Proliferation gemessen. Die Ergebnisse zeigen deutlich, daß die TCC mit den Phl p 5b-Mutanten spezifisch reagieren, wenn der entsprechende Epitop unverändert ist und zeigen erwartungsgemäß keine Reaktion, wenn der Epitop fehlt oder durch eine Punktmutation verändert ist.

### Abb. 7: Proliferative Reaktion von Phl p 5b-reaktiven T-Zell-Klonen (TCC) von Allergikern mit rPhl p 5b-Mutanten

**Spezifität: Immundominanter T-Zell-reaktiver Bereich A**

| | | **[**^{**3**}**H]-Thymidineinbau in TCC**^{**2)**} | | | |
|---|---|---|---|---|---|
| Stimulätor¹⁾ | Epitop vorhanden | JR 15 | JR 13 | CB 14 | CB 2 |
| Medium Medium | - | - | - | - | - |
| n Phl p5 | + | +++ | +++ | +++ | +++ |
| r Phl p5a | (±) | - | - | + | + |
| r Phl p5b | + | +++ | +++ | +++ | +++ |
| PM1 | + | ++ | +++ | +++ | +++ |
| PM3 | + | +++ | +++ | +++ | +++ |
| DM1 | + | +++ | +++ | +++ | +++ |
| DM2 | + | +++ | +++ | ng³⁾ | ng |
| DM2* | - | - | - | - | - |
| DM3 | + | +++ | +++ | ng | ng |
| PL(12mer) | + | +++ | +++ | +++ | +++ |

| | | | | | |
|---|---|---|---|---|---|
| ¹ Endkonzentration 0.3 µM | | | | | |
| ² Stimulationsindex SI: < 1(-), 1-2 (+), 2-5(+), 5-10 (++), > 10 (+++) | | | | | |
| ³n.g.: nicht getestet | | | | | |

### Abb. 8: Proliferative Reaktion von Phl p 5b-reaktiven T-Zell-Klonen (TCC) von Allergikern mit rPhl p 5b-Mutanten

**Spezifität: Immundominanter T-Zell-reaktiver Bereich B**

| | | **[**^{**3**}**H]-Thymidineinbau**^{**2)**} **in TCC** | |
|---|---|---|---|
| Stimulator¹⁾ | Epitop vorhanden | UZH2 | DW8 |
| Medium | - | - | - |
| n Phl p5 | + | +++ | +++ |
| r Phl p5a | (±) | ± | +++ |
| r Phl p5b | + | +++ | + |
| PM1 | + | +++ | + |
| PM3 | + | +++ | ± |
| DM1 | + | +++ | + |
| DM2 | - | - | - |
| DM2* | - | - | - |
| DM3 | + | +++ | + |
| PL (12mer) | + | +++ | +++ |

| | | | |
|---|---|---|---|
| ¹ Endkonzentration 0.3 µM | | | |
| ² Stimulationsindex Sl: < 1(-), 1-2 (+), 2-5(+), 5-10 (++), > 10 (+++) | | | |
| ³ n.g.: nicht getestet | | | |

### Abb. 9: Proliferative Reaktion des Phl p 5b-reaktiven T-Zell-Klonen (TCC) von Allergikern mit rPhl p 5b-Mutanten

**Spezifität: Immundominanter T-Zell-reaktiver Bereich C**

| | | **[**^{**3**}**H]-Thymidineinbau**^{**2)**} | | | |
|---|---|---|---|---|---|
| Stimulator¹⁾ | Epitop unverändert vorhanden | 1. Exp. | 2. Exp. | 3. Exp. | |
| Medium | - | 1 | 1 | 1 | - |
| n Phl p5 | + | 11,2 | 8,2 | 4,5 | ++ |
| r Phl p5a | - | ng | <1 | <1 | - |
| r Phl p5b | + | 11,0 | 7,0 | 5,5 | ++ |
| PM1 | - | <1 | <1 | 1,1 | - |
| PM3 | + | 7,4 | 5,9 | 4,5 | ++ |
| DM1 | + | 8,6 | 6,2 | 4,4 | ++ |
| DM2 | + | 14,4 | 9,1 | 7,1 | +++ |
| DM2* | + | 12,8 | 12,1 | 11,7 | +++ |
| DM3 | + | 9,8 | 6,9 | 4,4 | ++ |
| PL(12mer) | + | 20,9 | 16,7 | ng³⁾ | +++ |

| | | | | | |
|---|---|---|---|---|---|
| ¹ Endkonzentration 0,3 µM | | | | | |
| ² Stimulationsindex Sl: < 1(-), 1-2 (+), 2-5(+), 5-10 (++), > 10 (+++) | | | | | |
| ³ n.g.: nicht getestet | | | | | |

### Beispiel 7

### Testung der Reaktivität von rekombinanten Phl p 5b-Mutanten mit T-Zell-Linien von Graspollenallergikern

Von 8 Graspollenallergikern (s. Bsp. 1) wurden die oligoklonalen T-Zell-Linien (TCL) durch wiederholte Aktivierung mit natürlichem Phl p 5b (a + b) oder rekombinantem rPhl p 5b bzw. 5a + 5b angelegt.

Diese TCL wurden mit den rPhl p 5b-Mutanten auf ihre proliferative Reaktion getestet (Abb. 10). Dabei zeigt sich, daß alle Mutanten die TCL aktivieren, wobei jedoch quantitative Unterschiede bestehen. Die Deletionsmutante DM3 zeigt bei den meisten TCL eine starke spezifische Stimulierung.

### Abb. 10: Proliferative Reaktion des Phl p 5b-reaktiven T-Zell-Linien (TCL) von Allergikern mit rPhl p 5b-Mutanten

| | **[**^{**3**}**H]-Thymidineinban in TCL**^{**2)**} | | | | | | | |
|---|---|---|---|---|---|---|---|---|
| **TCL** | **1 Wöl** | **2 Eic** | **3 Fre** | **4 Mer** | **6 Mah** | **5 17.4** | **7 19.2** | **8 20.1** |
| Primärer Stimulator | n Phl p 5 | n Phl p 5 | n Phl p 5 | n Phl p 5 | rPhl p 5a | r Phl p 5 | r Phl p 5b | r Phl p 5b |
| | | | | | r Phl p 5b | | | |
| Sekundärer Aktivator¹⁾ | | | | | | | | |
| n Phl p 5 | +++ | +++ | ++ | +++ | +++ | + | +++ | + |
| r Phl p 5a 5a | - | + | + | + | ++ | ng³⁾ | ng | ng |
| r Phl 5b Phl 5b | + | + | + | + | +++ | + | +++ | +++ |
| PM1 | + | ± | + | ± | ++ | + | +++ + | +++ |
| PM3 | ± | ± | + | + | ± | + | +++ | + |
| DM1 | ± | + | + | + | ++ | + | +++ | +++ |
| DM2 | ± | + | +++ | +++ | ++ | + | ++ | +++ |
| DM2* | ± | + | + | ++ | ++ | + | + | ± |
| DM3 | ± | + | ++++ + | ++ | +++ | ++ | +++ | +++ |

| | | | | | | | | |
|---|---|---|---|---|---|---|---|---|
| ¹ Endkonzentration 0.3 µM | | | | | | | | |
| ² Stimulationsindex Sl: < 1(-), 1-2 (+), 2-5(+), 5-10 (++), > 10 (+++) | | | | | | | | |
| ³ n.g.: nicht getestet | | | | | | | | |

### Zusammenfassende Beurteilung der Ergebnisse gemäß der Beispiele 1 - 7

Die Kartierung der von den T-Helferzellen von Graspollen-Allergikern erkannten Epitope des Phl p 5b-Hauptallergens hat gezeigt, daß die T-Zell-Epitope der individuellen T-Zell-Klone (TCL) über die gesamte Sequenz des Phl p 5b verteilt sind. Es lassen sich jedoch unschwer **3 immundominante T-Zell-reaktive Bereiche** erkennen, die von 85% der TCC erkannt werden (Beispiel 1). Durch Punktmutationen (Beispiel 2) und Deletionsmutationen (Beispiel 3) konnten rekombinante Phl p 5b-Mutanten erzeugt werden. Die Punktmutanten (PM1 und PM3) unterscheiden sich hinsichtlich ihrer lgE-Reaktivität, gemessen im EAST-Hemmtest (Beispiel 4), nur unwesentlich vom Wildtyp Phl p 5b. Die lgE-Reaktivität der Deletionsmustanten DM1 und DM3 ist stark reduziert, aber noch nachweisbar. Demgegenüber ist die lgE-Bindung der Mutanten DM2 und DM2* extrem stark reduziert. Diese graduelle Abnahme der Allergenität der rPhl p 5b-Mutanten wird auch durch den Histamin-Freisetzungstest mit Spez. lgE beladenen Basophilen aus dem Blut von Allergikern bestätigt (Beispiel 5). Die Prüfung der rPhl p 5b-Mutanten mit Epitop-kartierten T-Zell-Klonen bestätigt, daß die Punkt- und Deletionsmutationen in der erwarteten Weise mit dem TCC reagieren oder die Stimulierung nicht erfolgt (Beispiel 6). An oligoklonalen T-Zell-Linien, die aus dem Blut von Graspollenallergikern durch Stimulierung mit Phl p 5 angelegt wurden, konnte gezeigt werden, daß die Mutanten zu einer Stimulierung solcher oligoklonalen TCL fähig sind (Beispiel 7). Faßt man die Ergebnisse der Reduzierung der Allergenität und den Erhalt der T-Zell-Stimulierung zusammen, so stellen die Mutanten, besonders die Deletionsmutanten, rekombinante Allergenvarianten dar, die prospektiv zur spezifischen Immuntherapie geeignet sind.

## Patentansprüche

1. Verfahren zur Identifizierung modifizierter rekombinanter Graminaenpollen-Allergene, die von den Hauptgruppen 1 oder 5 abgeleitet sind, deren Reaktivität mit lgE-Antikörpern von gegen diese Allergene allergischen Patienten eliminiert oder reduziert ist, wobei die Reaktivität mit T-Lymphozyten weiterhin erhalten ist, **dadurch gekennzeichnet, daß** ausgehend von der Aminosäuresequenz des natürlichen Protein-Allergens
- eine Reihe von überlappenden Oligopeptiden, die von der Sequenz des Allergens abgeleitet sind, hergestellt werden,
- T-Zell-Klone von Patienten, die gegen besagtes Allergen allergisch sind, etabliert werden,
- besagte Oligopeptide auf ihre Fähigkeit, mit den T-Zell-Klonen zu reagieren und diese zu stimulieren, getestet werden,
- Mutanten des besagten Allergens hergestellt werden, wobei einer oder mehrere der T-Zell reaktiven Sequenzbereiche (T-Zell-Epitope) unverändert gelassen werden,
- diejenigen Mutanten identifiziert werden, die die angestrebten Eigenschaften aufweisen.

## Claims

1. Process for the identification of modified recombinant Graminae pollen allergens which are derived from main group 1 or 5 and whose reactivity with IgE antibodies from patients who are allergic to these allergens has been eliminated or reduced, while the reactivity with T lymphocytes is maintained, **characterised in that**, starting from the amino acid sequence of the natural protein allergen,
- a series of overlapping oligopeptides derived from the sequence of the allergen is prepared,
- T-cell clones from patients who are allergic to the said allergen are established,
- the said oligopeptides are tested for their ability to react with the T-cell clones and to stimulate the latter,
- mutants of the said allergen are prepared, with one or more of the T-cell-reactive sequence regions (T-cell epitopes) being left unchanged,
- the mutants which have the target properties are identified.

## Revendications

1. Procédé pour l'identification d'allergènes de pollen de graminée recombinés modifiés qui sont dérivés à partir d'un groupe principal 1 ou 5 et dont la réactivité avec des anticorps IgE issus de patients qui sont allergiques à ces allergènes a été éliminée ou réduite, tandis que la réactivité avec des lymphocytes T est maintenue, **caractérisé en ce que**, en partant de la séquence des acides aminés de l'allergène de protéine naturel,
- une série d'oligopeptides en chevauchement qui sont dérivés à partir de la séquence de l'allergène est préparée,
- des clones de cellules T issus de patients qui sont allergiques audit allergène sont établis,
- lesdits oligopeptides sont testés quant à leur capacité à réagir avec les clones de cellules T et à stimuler ces derniers,
- des mutants dudit allergène sont préparés, une ou plusieurs des régions de séquence réactives aux cellules T (épitopes de cellules T) étant laissées inchangées,
- les mutants qui présentent les propriétés cibles sont identifiés.
